Europäisches Patentamt

European Patent Office

Office européen des brevets

(11) Publication number: **0 141 915**
**B1**

(12) **EUROPEAN PATENT SPECIFICATION**

(45) Date of publication of patent specification: **13.06.90**

(51) Int. Cl.⁵: **A 61 N 2/10**

(21) Application number: **84109186.1**

(22) Date of filing: **02.08.84**

(54) Plug for use in human medicine.

(30) Priority: **02.08.83 YU 1608/83**

(43) Date of publication of application:
**22.05.85 Bulletin 85/21**

(45) Publication of the grant of the patent:
**13.06.90 Bulletin 90/24**

(84) Designated Contracting States:
**AT BE CH DE FR GB IT LI LU NL SE**

(56) References cited:
**DE-A-2 502 164**
**DE-A-2 822 616**
**DE-A-3 041 965**
**DE-A-3 327 086**
**FR-A-2 329 300**
**US-A-3 650 275**
**US-A-3 800 800**

**SOVIET INVENTIONS ILLUSTRATED, Section P,
Week B49, January 23, 1980DERWENT
PUBLICATIONS LTD., London, P34**

(73) Proprietor: **Dekleva, Nikola, Ph.D., M.D.**
**Otona Zupancica 27**
**Novi Beograd (YU)**
(73) Proprietor: **Majic, Vidan, Ph.D., Dipl.-Ing.**
**Bulevar Revolucije 249**
**Beograd (YU)**
(73) Proprietor: **Belesnin, Bogdan, Prof., Ph.D., M.D.**
**Vojvode Stepe 141**
**Beograd (YU)**
(73) Proprietor: **Antic, Radoslav, Dipl.-Ing.**
**Ljermontova 6/121**
**Beograd (YU)**

(72) Inventor: **Dekleva, Nikola, Ph.D., M.D.**
**Otona Zupancica 27**
**Novi Beograd (YU)**
Inventor: **Majic, Vidan, Ph.D., Dipl.-Ing.**
**Bulevar Revolucije 249**
**Beograd (YU)**
Inventor: **Belesnin, Bogdan, Prof., Ph.D., M.D.**
**Vojvode Stepe 141**
**Beograd (YU)**
Inventor: **Antic, Radoslav, Dipl.-Ing.**
**Ljermontova 6/121**
**Beograd (YU)**

Courier Press, Leamington Spa, England.

# EP 0 141 915 B1

74 Representative: **Lehn, Werner, Dipl.-Ing. et al
Hoffmann, Eitle & Partner Patentanwälte
Arabellastrasse 4
D-8000 München 81 (DE)**

## Description

The present invention relates to a plug for insertion into a human bodily opening and comprising an elongate insertion member having a smooth, rounded outer surface and an entry-limiting member at one end of the insertion member.

Such a plug is known from DE-A-25 02 164. In this document, the plug is provided with external metal electrodes for application of an electrical voltage to cause contraction of the anal sphincter muscle. Furthermore, a central axially extending tube is provided for administering a barium enema.

DE-A-30 41 965 discloses the application of permanent magnets to physiotherapy. The magnets are contained in a large flat housing on which the patient is expected to lie for treatment of limbs, abdomen or neck etc. A plurality of permanent magnetic elements is provided having a predetermined distance with each other which depends on the nerves to be excited or energized by considering the speed of the blood pulsation wave in a blood vessel. The respective distance between the permanent magnets considers also a certain time period in which the nerves as stimulated are not to be stimulated by another stimulus.

The present invention is applicable in the field of magnetobiology, biomagnetism and magnetotherapy in the broader sense, and in a narrower sense in rectal and vaginal therapy - especially in cases of haemorrhoid treatment.

Inflammatory processes in rectal and vaginal openings are treated classically by the aid of injections, liquid medicines, ointments or suppositories.

In cases of haemorrhoids, surgical methods such as ligation cauterization and others have been applied. The classical way of treatment is relatively long, and the proceedings are impeded owing to frequent resistance to medicines.

History records controlled magnetotherapy as early as 200 B.C. Magnetism was used in healing various health disorders. The achieved success attributed to magnets the property of being universal healer, as well as a guarantee of immortality. In ancient times, magnets were used to cure headaches, spasms, nerve disorders, ulcers, bleeding sicknesses and hydropsy.

In spite of that, magnetic fields were long neglected and even forgotten in medicine.

But in the second half of the XIX century mankind turned to magnetotherapy again. Nevertheless, the most valuable investigating results fall into the last decade (L. Hallagren: Inductive neural stimulator, IEEE Trans. on BME, 470-472, Nov. 1973., B. Beleslin, N. Dekleva, V. Majić: Some possibilities in electro-magnetic tissue stimulation, bioeng 80, London, 111-112, 1980).

One of the fundamental investigation results of magnetobiological effect is the magnetic field action on leech ganglion cells giving electrical spike activity when subjected to magnetic fields (B. Beleslin, V. Majić: The effects of pulsed magnetic field on the nerve cells of leeches, Periodicum biologorum, 76, 39, 1974., V. Majić, B. Beleslin, B. Stamenović, N. Dekleva: Biomagnetism and artificial magnetic stimulation of living structures, XXIX congress of International Astronautical Federation, Dubrovnik, 1-9, 1978).

The influence of magnetic field on the heart action has been investigated, as well as spacial heart orientation in vitro according to the vector of magnetic flux density (B. Stamenović, V. Majić: Heart function in magnetic field, Periodicum biologorum, 77, 57-61, 1975).

Bactericidal action of magnetic field, bone healing in magnetic field, magnetotherapy in otorhinolaryngology, ophtalmology, and periodontal diseases have been known and accepted in clinical methods (S. Petronijević, N. Dekleva, B. Beleslin, V. Majić: Magnetotherapy in otorhinolaryngology, World congress on medical physics and biomedical engineering, Hamburg, 12.22, 1982, B. Stefonavić, B. Beleslin, N. Dekleva, V. Majić: Magnetostimulators in opthalmology, World Congress on medical physics and biomedical engineering, Hamburg, 12.23, 1982, A. Skokljev, N. Dekleva, B. Beleslin, V. Majić: Magnetostimulators in periodontal diseases treatment, Third Mediterranean Conference on Medical and Biological Engineering, Portoroz, 7.4, 1983).

As far as is known, the field of haemorrhoid reaction to magnetic fields has not been investigated and is previously unknown.

An object of the invention is to provide a simple and effective device for rectal and/or vaginal therapy, especially a therapeutic plug for insertion into a human bodily opening.

Based on a plug for insertion into a human bodily opening and comprising an elongate insertion member having a smooth, rounded outer surface and an entry-limiting member at one end of the insertion member, the object of the invention is solved in that said insertion member comprises permanent magnetic means comprising at least one permanent magnetic element providing a plurality of magnetic pole-pairs (N, S) with a magnetic flux density not greater than 80mT.

For a better understanding of the invention, and to show how the same may be carried into effect, reference will now be made, by way of example, to the accompanying drawings, in which:

Fig. 1 shows an end view (left) and a side view (right) of a plug according to one embodiment of the invention;

Figs. 2 to 5 show configurations of plug according to four further embodiments of the invention; and

Figs. 6 to 10 show five further embodiments of the invention having different arrangements of magnetic pole-pairs in a plug having the configuration shown in Fig. 1.

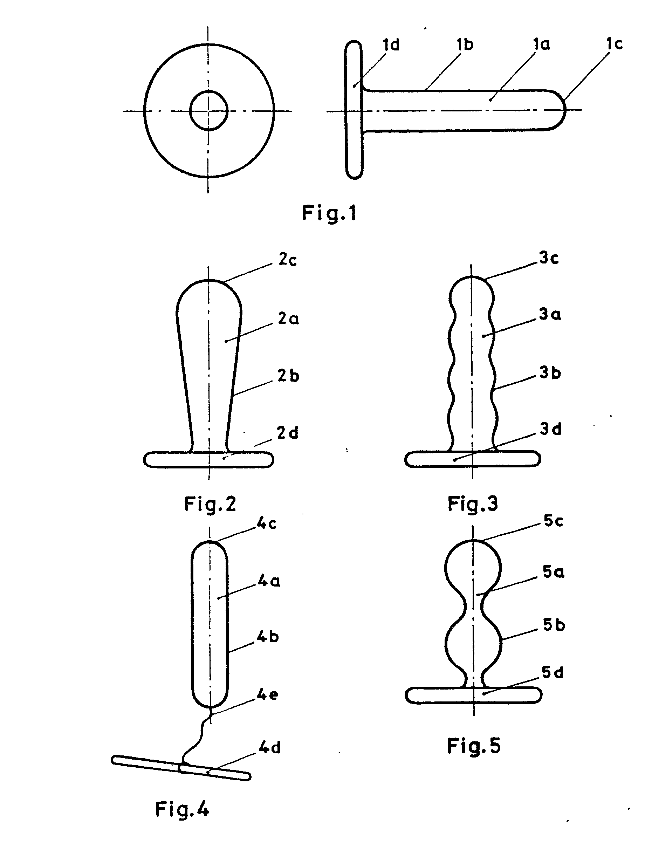

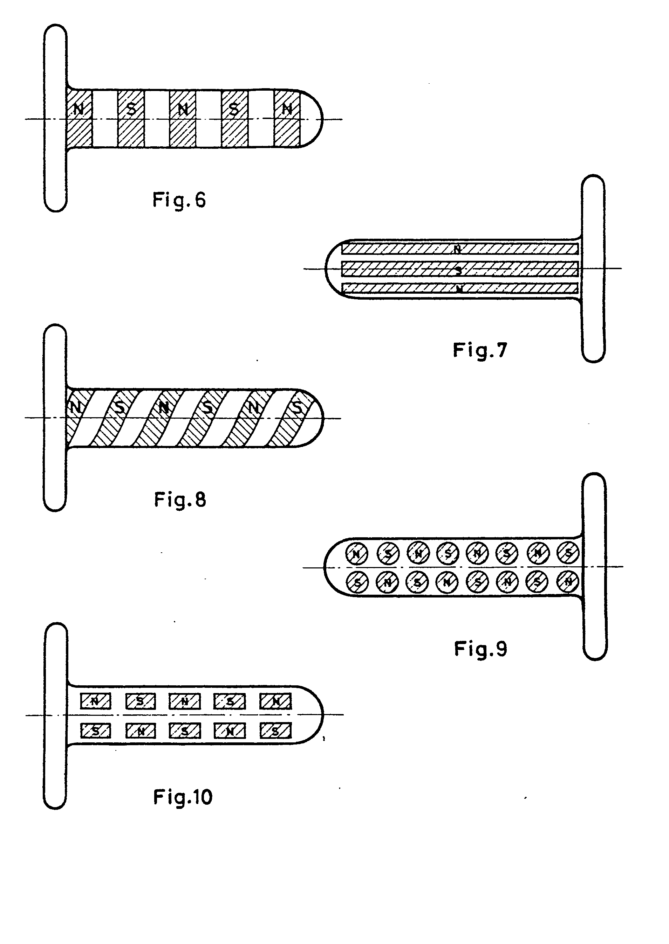

Of course, the configurations of magnetic pole-pairs in Figs. 6 to 10 may also be employed in the embodiments of Figs. 2 to 5.

Referring to the Figures in more detail, each of Figures 1 to 5 shows a respective embodiment of magnetotherapeutic plug having an elongate insertion member 1a, 2a, 3a, 4a or 5a and an entry-limiting member 1d, 2d, 3d, 4d or 5d at one end thereof. The insertion member has in each case a rounded end 1c, 2c, 3c, 4c or 5c and is coated with biologically inactive material 1b, 2b, 3b, 4b or 5b. In Figure 1, the insertion member 1a is rod-like or cylindrical in form of circular or elliptical or oval cross-section, whilst Figure 2 shows a thickened end and tapering shank. Figure 3 shows an undulating surface, and Figure 5 shows a configuration approximating a dumbell or double ball shape.

In Figure 4, the insertion member 4a is similar to member 1a of Figure 1, but the limiting member 4d is separate and attached to member 4a by a thread 4e.

Of course, the thread 4e should also be of biologically inactive material and preferably the entry-limiting member in each of Figures 1 to 10 is also coated with or made of biologically inactive material.

Figures 6 to 10 show various configurations of permanent magnetic pole-pairs which may be employed. In these Figures, any outer coating of biologically inactive material is assumed to be removed to expose the permanent magnetic core the pole regions of which are in each case indicated by shading. The polarity is indicated by N (north pole) or S (south pole) as necessary.

In Figures 6, 7, 9 and 10 a plurality of individual magnetic pole regions are provided, whilst in Figure 8 continuous helical regions are provided. Figure 6 shows circular rings, Figure 7 shows longitudinal bands, Figure 9 shows small circular regions, and Figure 10 rectangular regions.

The magnetic pole configuration may thus be axial, radial, helical, circular, rectangular or any other geometrical shape.

The magnetic core may be of alnico alloy, of hard sintered or plastic ferrite material of barium or strontium type, or of metals from the rare-earth group. The magnetic flux density on the surface of the magnetically active part of the plug should not exceed 80 mT and is preferably in the range of from 20 mT to 40 mT.

Now the use of the above described magnetotherapeutic plug will be explained.

A magnetotherapeutic tool for healing, aimed for broad use, is provided by the magnetotherapeutic plug. The plug is for insertion in a bodily opening such as the rectal or vaginal cavity.

The most convenient shape of the magnetotherapeutic plug is a rod of circular, elliptical or any other cross-section with rounded edges, to avoid mechanical damage of the tissue. For that purpose, the outer plug surfaces may be coated by a smooth layer of non-magnetic and biologically inactive material, deposited by one of the usual procedures, or formed in the shape of a separate capsule in which the magnetic core is inserted.

To provide the most suitable position of the plug in the rectal or vaginal opening (ostium), as well as easy extraction after use, one end of the plug is formed in the shape of a limiter, circular or any other shape, made of non-magnetic material.

The body of the plug can also be of unequal thickness, with a frontal thickening or more thickenings with undulating shape, aimed at preventing unwanted falling out of the magnetotherapeutic plug from the rectal or vaginal opening during use. Besides the shape of a rod with a limiter, shown in Figures 1 and 2, the plug can have the shape of a small ball or capsule with a thread and the limiter on the other side. Another convenient shape has small double balls, the ball having the limiter, remaining out during the treatment.

In our preliminary and prolonged clinical investigations, very good results have been obtained in the treatment of external and internal haemorrhoids by the aid of magnetotherapeutic plugs. After the second application, pain relief can be registered, and after the fourth one it vanishes. Besides, mucus discharge causing pruritus ani and faecal soiling also diminishes. Nevertheless, the procedure is repeated until the tenth treatment, lasting altogether ten days. This procedure preferably includes the alternate application of plugs with mutually different magnetic pole configurations.

Daily stimulation may last from one to four hours.

The plugs can be delivered in sterile packing, in which case the plugs are discarded after use. But the patients with modest financial possibilities can use it repeatedly with compulsory washing after every use and in the same way the active clyster part is washed after use. The plugs should be washed with warm water and soap or mild detergent solution and rinsed afterwards.

Before the application, the dry active part of the plug must be coated with medical petroleum jelly e.g. vaseline® and after that squeezed into the rectal or vaginal opening till the limiter.

The plug may be applied in a daily treatment of from 1 to 4 hours over a period of 7 to 14 days, preferably 10 days. Plugs having different geometric pole configurations may preferably be used alternately.

Twenty years at investigations and practical experience in the field of magnetobiology, biomagnetism and magnetotherapy lead the present inventors to feel justified in advocating use of electromagnets at clinics and hospitals as well as the use of permanent magnets in individual-home therapies, although the effect of varying electromagnetic field is stronger and faster. Electromagnets are unsuitable for home use for safety reasons. Permanent magnets have no power supplies which, regardless of all the precaution, can provoke injuries, with even fatal results.

However, in clinical practice, time varying, pulse monopolar and bipolar magnetic fields, with or without pulsing with or without amplitude modulation and variously shaped signals have been used. But all magnetotherapies of this kind involve the use of electro-magnetic stimulators with internal or external power supplies. Therefore, the safest and simplest magnetotherapy to apply by non-professional people, suffering from haemorrhoids, is the one with magnetostimulators made of permanent magnets.

Thus, by the aid of magnetic field, it is possible to attain soothing the inflammatory processes effectively and quickly. Magnetotherapy is performed by application of a plug, whose core is a magnetostimulator.

**Claims**

1. A plug for insertion into a human bodily opening and comprising an elongate insertion member (la, 2a, 3a, 4a, 5a) having a smooth, rounded outer surface and an entry-limiting member (1d, 2d, 3d, 4d, 5d) at one end of the insertion member, characterized in that said insertion member comprises permanent magnet means comprising at least one permanent magnetic element providing a plurality of magnetic pole-pairs (N, S) with a magnetic flux density not greater than 80mT.

2. A plug according to claim 1, characterized in that said smooth rounded outer surface is of biologically inactive material.

3. A plug according to claim 1 or 2, characterized in that at least the insertion member is constituted of biologically inactive material.

4. A plug according to claim 1 or 2, characterized in that the insertion member is at least partially coated with biologically inactive material (Ib, 2b, 3b, 4b, 5b).

5. A plug according to any one of claims 1 to 4, characterized in that the entry-limiting member is attached to said insertion member by an elongate flexible element such as a thread (4e).

6. A plug according to any one of claims 1 to 5, characterized in that the insertion member includes at least one portion of increased diameter (Figs. 2, 3 and 5).

7. A plug according to claim 6, characterized in that the insertion member comprises at least one substantially spherical portion (Fig. 5).

8. A plug according to any one of claims 1 to 7, characterized in that said magnet means comprises a magnetic core magnetized to provide a plurality of pole regions.

9. A plug according to claim 8, characterized in that each magnetic pole region has the shape of an elongate axial strip, a rectangular zone, a circular zone, an annular strip or a helical strip.

10. A plug according to any one of the preceding claims, characterized in that said at least one magnetic element is of alnico alloy, hard or plastic ferrite material of barium or strontium type or rare-earth metals.

11. A plug according to any one of the preceding claims characterized in that the magnetic flux density of the insertion member is in the range 20mT to 40mT.

**Patentansprüche**

1. Stöpsel zum Einsetzen in eine Öffnung des menschlichen Körpers, umfassend ein längliches Einsetzglied (1a, 2a, 3a, 4a, 5a) mit einer glatten, gerundeten Außenfläche und ein die Einführung begrenzendes Glied (1d, 2d, 3d, 4d, 5d) an einem Ende des Einsetzgliedes, dadurch gekennzeichnet, daß das Einsetzglied eine Permanentmagnet-Einrichtung umfaßt, welche wenigstens ein permanent-magnetisches Element enthält, das mehrere magnetische Polpaare (N, S) bildet mit einer magnetischen Flußdichte nicht größer als 80 mT.

2. Stöpsel nach Anspruch 1, dadurch gekennzeichnet, daß die besagte glatte gerundete Außenfläche aus biologisch inaktivem Material ist.

3. Stöpsel nach Anspruch 1 oder 2, dadurch gekennzeichnet, daß wenigstens das Einsetzglied aus biologisch inaktivem Material ist.

4. Stöpsel nach Anspruch 1 oder 2, dadurch gekennzeichnet, daß das Einsetzglied wenigstens teilweise mit biologisch inaktivem Material (1b, 2b, 3b, 4b, 5b) beschichtet ist.

5. Stöpsel nach einem der Ansprüche 1 bis 4, dadurch gekennzeichnet, daß das die Einführung begrenzende Glied mit dem Einsetzglied durch ein längliches flexibles Element wie zum Beispiel einen Faden (4e) verbunden ist.

6. Stöpsel nach einem der Ansprüche 1 bis 5, dadurch gekennzeichnet, daß das Einsetzglied wenigstens einen Abschnitt größeren Durchmessers aufweist (Fig. 2, 3 und 5).

7. Stöpsel nach Anspruch 6, dadurch gekennzeichnet, daß das Einsetzglied wenigstens einen im wesentlichen sphärischen Abschnitt aufweist (Fig. 5).

8. Stöpsel nach einem der Ansprüche 1 bis 7, dadurch gekennzeichnet, daß die besagte Magnet-Einrichtung einen Magnetkern aufweist, der magnetisiert ist zur Bildung von mehreren Polbereichen.

9. Stöpsel nach Anspruch 8, dadurch gekennzeichnet, daß jeder magnetische Polbereich die Gestalt eines länglichen axialen Streifens, einer rechteckigen Zone, einer kreisförmigen Zone, eines ringförmigen Streifens oder eines schraubenförmigen Streifens aufweist.

10. Stöpsel nach einem der vorangehenden Ansprüche, dadurch gekennzeichnet, daß wenigstens ein magnetisches Element aus einer Alnico-Legierung, hartem oder plastischem Ferritmaterial vom Barium- oder Strontiumtyp oder aus einem Metall der seltenen Erden ist.

11. Stöpsel nach einem der vorangehenden Ansprüche, dadurch gekennzeichnet, daß die magnetische Flußdichte des Einsetzgliedes im Bereich von 20 mT bis 40 mT liegt.

**Revendications**

1. Tampon destiné a être inséré dans un orifice du corps humain et comprenant un élément d'insertion allongé (1a, 2a, 3a, 4a, 5a) présentant une surface externe arrondie lisse et un élément de limitation d'entrée (1d, 2d, 3d, 4d, 5d) à une extrémité de l'élément d'insertion, caractérisé en ce que ledit élément d'insertion comprend un moyen d'aimant permanent comprenant au moins un élément magnétique permanent fournissant une pluralité de paires de pôles magnétiques (N, S) avec une densité de flux magnétique qui ne dépasse pas 80 mT.

2. Tampon selon la revendication 1, caractérisé en ce que ladite surface externe arrondie lisse est constituée par un matériau inactif du point de vue biologique.

3. Tampon selon la revendication 1 ou 2, caractérisé en ce qu'au moins l'élément d'insertion est constitué par un matériau inactif du point de vue biologique.

4. Tampon selon la revendication 1 ou 2, caractérisé en ce que l'élément d'insertion est au moins partiellement revêtu d'un matériau inactif du point de vue biologique (1b, 2b, 3b, 4b, 5b).

5. Tampon selon l'une des revendications 1 à 4, caractérisé en ce que l'élément de limitation d'entrée est fixé audit élément d'insertion par un élément flexible allongé tel qu'un fil (4e).

6. Tampon selon l'une des revendications 1 à 5, caractérisé en ce que l'élément d'insertion comprend au moins une partie de diamètre accru (figures 2, 3 et 5).

7. Tampon selon la revendication 6, caractérisé en ce que l'élément d'insertion comprend au moins une partie sensiblement sphérique (figure 5).

8. Tampon selon l'une des revendications 1 à 7, caractérisé en ce que ledit moyen d'aimant comprend un noyau magnétique aimanté de façon à former une pluralité de régions polaires.

9. Tampon selon la revendication 8, caractérisé en ce que chaque région de pôle magnétique présente la forme d'une bande axiale allongée, d'une zone rectangulaire, d'une zone circulaire, d'une bande annulaire ou d'une bande hélicoïdale.

10. Tampon selon l'une des revendications précédentes, caractérisé en ce que ledit élément magnétique, dont il existe au moins un, est en alliage alnico, en matériau de ferrite dur ou plastique du type du baryum ou du strontium ou des éléments des terres rares.

11. Tampon selon l'une des revendications précédentes, caractérisé en ce que la densité de flux magnétique de l'élément d'insertion est comprise dans la gamme de 20 à 40 mT.

EP 0 141 915 B1

Fig.1

Fig.2

Fig.3

Fig.4

Fig.5

1

Fig. 6

Fig. 7

Fig. 8

Fig. 9

Fig. 10